# EUROPÄISCHE PATENTANMELDUNG

(11) **EP 2 425 734 A1**
(43) Veröffentlichungstag der Anmeldung: **07.03.2012**
(21) Anmeldenummer: 10405163.6
(22) Anmeldetag: 03.09.2010
(51) Int. Cl.: A43D 1/02, A61B 5/107

(54) **Vorrichtung zur Herstellung eines Abdrucks eines Fusses**

(71) Anmelder: Lobster Shoes AG, 8002 Zürich (CH)
(72) Erfinder: Karaarslan, Cemal, 8001 Zürich (CH)
(74) Vertreter: Rüfenacht, Philipp Michael

(57) **Zusammenfassung**

Die Erfindung betrifft eine Vorrichtung (10) zur Herstellung eines Abdrucks eines Fusses mit einer Aufnahme für den Fuss mit wenigstens zwei zueinander schwenkbar gelagerten Teilen (1, 2, 3, 4). Die Aufnahme ist durch Verschwenken in eine erste Konfiguration und in eine zweite Konfiguration bringbar. In der zweiten Konfiguration sind die wenigstens zwei Teile (1, 2, 3, 4) derart angeordnet, dass die Vorrichtung (10) eine kistenähnliche Form aufweist und sich Innenseiten der wenigstens zwei Teile (1, 2, 3, 4) in einem inneren Bereich der kistenähnlichen Form der Vorrichtung befinden. Durch eine Überführung der Vorrichtung (10) von der ersten Konfiguration in die zweite Konfiguration ist ein Fuss in den inneren Bereich der kistenähnlichen Form der Vorrichtung bringbar. Durch eine Überführung der Vorrichtung (10) von der zweiten Konfiguration in die erste Konfiguration ist der Fuss aus der kistenähnlichen Form der Vorrichtung (10) in der zweiten Konfiguration befreibar.

## Beschreibung

Die Erfindung betrifft eine Vorrichtung und ein Verfahren zur Herstellung eines Abdrucks eines Fusses.

### Stand der Technik

Um einen Leisten für die Anfertigung eines Schuhs herzustellen oder einen bestehenden Leisten anzupassen, nimmt der Schuhmacher am Fuss manuell die Masse wie z. B. die Fusslänge, die Fussbreite oder Fussweite. Das kann beispielsweise mit einer Schablone erfolgen, bei welcher die Ferse an einem festen Anschlag bündig platziert wird und anschliessend ein verstellbarer Anschlag bündig dem vordersten Zeh eingestellt wird. Über eine Messskala kann anschliessend die Fusslänge schnell und einfach abgelesen werden. Mit einer entsprechenden Messmethode lässt sich auch die Fussbreite oder Fussweite ausmessen.

Die GB 943586 zeigt das Herstellen eines Abdrucks eines Fusses, wobei der Fuss in ein Bett gepresst wird, welches mit kleinen Plastikbällchen gefüllt und mit einer luftdichten Gummimatte abgedeckt ist, so dass der Abdruck erhalten bleibt. Diese Vorrichtung ist auch als "orthopädische Box" bekannt, welche auch als Behälter mit einem Hartschaum ausgeführt sein kann, mit welchem ein Sohlenabdruck für Einlagen oder Ähnliches abgenommen werden kann.

Die US 5,364,580 zeigt ein Körperabdrucksystem, bei welchem ein Körperteil wie z. B. das Bein durch einen Schlitz in eine Form geführt wird. Der Schlitz wird verschlossen und eine gelatineartige Flüssigkeit wird in die Form gegossen. Der Körperteil wird aus der Form entfernt und anschliessend wird mit dem aus der Gelatinemasse bestehenden Negativ ein Abguss des Körperteils erstellt.

Es ist auch bekannt, den Fuss mit einem 3D-Scanner direkt auszumessen, um anhand der Messdaten Artikel herzustellen.

Bei einer manuellen Messmethode können aus Zeitgründen und insbesondere mangels klar definierter Referenzpunkte am Fuss nur eine beschränkte Anzahl Messpunkte erfasst werden. Der daraus hergestellte Leisten ist deshalb nur eine grobe Annäherung an den Fuss. Mit den im Stand der Technik bekannten Vorrichtungen können Fussabdrücke nur teilweise erstellt werden oder erst nach einem komplizierten Herstellungsverfahren. Mit einem 3D-Scanner kann zwar der Fuss exakt ausgemessen werden, wobei jedoch eine aufwändige Vorrichtung notwendig ist, welche nicht vor Ort zu Kunden transportiert werden kann und der Kunde somit das Haus verlassen muss, um einen 3D-Scan durchzuführen.

### Darstellung der Erfindung

Aufgabe der Erfindung ist es, eine dem eingangs genannten technischen Gebiet zugehörende Vorrichtung zu schaffen, um mit einer leicht transportierbaren Vorrichtung einen Abdruck eines Fusses schnell und exakt herzustellen.

Die Lösung der Aufgabe ist durch die Merkmale des Anspruchs 1 definiert. Die erfindungsgemässe Vorrichtung zur Herstellung eines Abdrucks eines Fusses zeichnet sich dadurch aus,
a) dass sie eine Aufnahme für den Fuss mit wenigstens zwei zueinander schwenkbar gelagerte Teile umfasst;
b) so dass die Aufnahme durch Verschwenken in eine erste Konfiguration und in eine zweite Konfiguration bringbar ist;
c) dass in der zweiten Konfiguration die wenigstens zwei Teile derart angeordnet sind, dass die Vorrichtung eine kistenähnliche Form aufweist und dass sich Innenseiten der wenigstens zwei Teile in einem inneren Bereich der kistenähnlichen Form der Vorrichtung befinden;
d) dass durch eine Überführung der Vorrichtung von der ersten Konfiguration in die zweite Konfiguration ein Fuss in den inneren Bereich der kistenähnlichen Form der Vorrichtung bringbar ist und
e) dass durch eine Überführung der Vorrichtung von der zweiten Konfiguration in die erste Konfiguration der Fuss aus der kistenähnlichen Form der Vorrichtung in der zweiten Konfiguration befreibar ist.

Ein oder mehrere der zueinander schwenkbare Teile weisen eine Öffnung für das Bein auf, so dass sich der Fuss in der zweiten Konfiguration im inneren Bereich der Vorrichtung befindet und andere Körperteile ausserhalb. Die Öffnung kann derart vorgesehen sein, dass Körperteile ab dem Knöchel, ab einer bestimmten Höhe des Unterschenkels oder erst oberhalb des Knies ausserhalb der Vorrichtung sind. Somit bietet die Öffnung Platz für einen Unterschenkel oder einen Oberschenkel, so dass sich in der zweiten Konfiguration der Fuss innerhalb der Vorrichtung befindet und der Unterschenkel oder der Oberschenkel durch die Öffnung hindurchgeführt ist. Der Innenraum der Vorrichtung ist eingerichtet, um einen Abdruck eines Fusses herzustellen. In der ersten Konfiguration kann der Fuss gegenüber der Vorrichtung derart platziert werden, dass sich der Fuss in der zweiten Konfiguration innerhalb der Vorrichtung befindet und somit ein Abdruck eines Fusses hergestellt werden kann.

Die kistenähnliche Form ist dahingehend zu verstehen, dass die Vorrichtung in der zweiten Konfiguration im Wesentlichen eine quaderförmige Form aufweist, wobei die Kanten rund oder eckig ausgeführt sein können. Ferner kann die kistenähnliche Form der Vorrichtung auch stufenartig angeordnete Abschnitte umfassen, wobei beispielsweise eine erste Stufe vorgesehen ist, um einen Teil des Unterschenkels und/oder des Knöchels aufzunehmen sowie eine zweite Stufe vorgesehen ist, um einen Teil der vorderen Fussbereiche wie der Zehen und/oder des Mittelfusses aufzunehmen.

Dadurch, dass sich der Fuss in der zweiten Konfiguration vollständig innerhalb des inneren Bereichs der Vorrichtung befindet, kann ein exakter Abdruck eines Fusses hergestellt werden. Indem die Teile schwenkbar gelagert sind, lassen sich diese rasch von der ersten Konfiguration in die zweite Konfiguration (und umgekehrt) bringen, womit eine schnelle Herstellung des Abdrucks eines Fusses gewährleistet ist. Durch die kistenförmige Form ist sichergestellt, dass sich die Vorrichtung leicht transportieren lässt.

Vorzugsweise ist durch eine Überführung der Vorrichtung von der ersten Konfiguration in die zweite Konfiguration der Fuss wenigstens bis zum Knöchel in den inneren Bereich der kistenähnlichen Form der Vorrichtung bringbar. Aus dem Abdruck eines Fusses lässt sich ein Leisten herstellen, welcher insbesondere für einen Halbschuh geeignet ist. Da nur der Fuss und der Knöchel umschlossen werden, ist die Abdrucksoberfläche im Vergleich zu der nachfolgend beschriebenen Alternative, bei welcher auch der Unterschenkel und das Knie umschlossen werden, relativ klein und die beiden Teile der Vorrichtung lassen sich mit einem verhältnismässig kleineren Kraftaufwand von der ersten Konfiguration in die zweite Konfiguration bringen.

Alternativ ist durch eine Überführung der Vorrichtung von der ersten Konfiguration in die zweite Konfiguration der Fuss wenigstens bis zum Knie in den inneren Bereich der kistenähnlichen Form der Vorrichtung bringbar. Aus dem Abdruck des Fusses lässt sich ein Leisten herstellen, welcher insbesondere für einen Stiefel geeignet ist. Da der Fuss, der Knöchel, der Unterschenkel und das Knie umschlossen werden, ist die Abdrucksoberfläche im Vergleich zu der vorgängig beschriebenen Variante, bei welcher nur der Fuss und der Knöchel umschlossen werden, relativ gross und die beiden Teile der Vorrichtung lassen sich nur mit einem verhältnismässig grösseren Kraftaufwand von der ersten Konfiguration in die zweite Konfiguration bringen.

Vorzugsweise ist die Vorrichtung formstabil ausgeführt, so dass sie in der zweiten Konfiguration sowohl mit als auch ohne Fuss im inneren Bereich der kistenähnlichen Form bei äusserer Krafteinwirkung ihre Form beibehält. Dadurch dass die Vorrichtung stabil ist, kann der Abdruck des Fusses sehr exakt hergestellt werden.

Alternativ ist die Vorrichtung nicht formstabil ausgeführt. Dadurch lässt sich die Vorrichtung kostengünstiger herstellen. Allerdings kann mit einer solchen nicht formstabilen Vorrichtung ein Abdruck eines Fusses weniger exakt hergestellt werden.

Die wenigstens zwei Teile können aus Karton, Kunststoff, Holz, Metallblech oder einer Kombination hiervon gefertigt sein. Je nach Anforderung an das Gewicht, an die Formstabilität oder an die Herstellungskosten der Vorrichtung können entsprechende Materialien gewählt werden. So ist eine aus Karton hergestellte Vorrichtung leicht und kostengünstig, jedoch nicht besonders formstabil. Mit Kunststoff lässt sich im Hinblick auf die Farbgebung oder die Form eine formstabile Vorrichtung kostengünstig herstellen. Eine aus Holz hergestellte Vorrichtung kann aus ästhetischen Gründen in einem Verkaufslokal besonders bevorzugt sein, während sich eine aus Metallblech hergestellte Vorrichtung durch eine hohe Formstabilität auszeichnen kann.

Vorzugsweise sind mindestens zwei der wenigstens zwei Teile auf der Innenseite mit einem Material zur Abnahme des Abdrucks eines Fusses ausgekleidet. Sobald die Vorrichtung in die zweite Konfiguration gebracht wird, entsteht in diesem Material der Abdruck des Fusses und bleibt in diesem Material bestehen, nachdem die Vorrichtung wieder in die erste Konfiguration gebracht wurde und der Fuss aus der Vorrichtung entfernt wurde.

Alternativ wird die Vorrichtung in die erste Konfiguration gebracht, dann werden das Material zur Abnahme des Abdrucks des Fusses und der Fuss zusammen angeordnet, sodass sich der Fuss und das Material innerhalb der Vorrichtung befinden, sobald die Vorrichtung in die zweite Konfiguration gebracht wird.

Bevorzugt ist das Material zur Abnahme des Abdrucks eines Fusses auf der Innenseite der mindestens zwei der wenigstens zwei Teile derart geformt, dass in der zweiten Konfiguration der Fuss lückenlos durch das Material umschliessbar ist. Durch das lückenlose Umschliessen wird sichergestellt, dass der Abdruck des Fusses vollständig ist und den gesamten Fuss umfasst.

Alternativ ist das Material zur Abnahme des Abdrucks eines Fusses derart geformt, dass Lücken bestehen bleiben. Das Material kann beispielsweise derart geformt sein, dass besonders wichtige Stellen wie z. B. die Ferse oder die Zehen lückenlos umschlossen sind, während an weniger wichtigen Stellen wie z. B. am Mittelfuss Lücken bestehen bleiben. Dadurch wird der Abdruck des Fusses nur unvollständig hergestellt, es kann jedoch der Komfort bei der Herstellung des Abdrucks eines Fusses verbessert werden.

Vorzugsweise ist das Material zur Abnahme des Abdrucks eines Fusses vollständig kompressibel und inelastisch, wobei es sich vorzugsweise um einen Hartschaum handelt. Wegen dem vollständig kompressiblen Material kann der Abdruck des Fusses ohne wesentliche Druckbelastung des Fusses hergestellt werden. Da das Material inelastisch ist, bleibt der Abdruck des Fusses exakt erhalten, auch nachdem die Vorrichtung von der zweiten in die erste Konfiguration übergeführt und der Fuss befreit ist.

Beispielsweise kann als Material zur Abnahme des Abdrucks eines Fusses der von der Spigron Spin GmbH, Gronau produzierte Trittschaum rosa, pink oder hellblau verwendet werden, für welchen das Zertifikat mit Identifkationsnummer 99200 des "Centrum für Hygiene und medizinische Produktsicherheit", ausgestellt in Schwerin am 25.06.2010, die biologische Unbedenklichkeit gemäss DIN EN ISO 10993-1 bestätigt.

Bevorzugt ist auf der Aufnahme und/oder dem Material zur Abnahme des Abdrucks eines Fusses eine Markierung für eine korrekte Positionierung des Fusses angebracht. Dadurch kann gewährleistet werden, dass bei der Herstellung des Abdrucks des Fusses keine Verletzungen entstehen und der Abdruck des Fusses vollständig hergestellt wird. Die zueinander schwenkbar gelagerten Teile können so gestaltet sein, dass möglichst wenige Möglichkeiten bestehen, um bei der Herstellung des Abdrucks eines Fusses Fehler zu machen. Auf der Vorrichtung kann eine Anleitung für den Gebrauch der Vorrichtung angebracht sein. Die Vorrichtung ist somit geeignet, um diese beispielsweise per Post einem Kunden zuzustellen, der mit der Vorrichtung den Abdruck des Fusses selbständig herstellen und die Vorrichtung zurückschicken kann, damit aufgrund des Abdrucks des Fusses ein Artikel wie ein Leisten und/oder ein massangefertigter Schuh hergestellt werden kann.

Vorzugsweise umfasst die Vorrichtung einen Mechanismus, welcher bei einer Überführung von der ersten Konfiguration in die zweite Konfiguration ein Erreichen der zweiten Konfiguration angibt, indem ein Einrasten spürbar oder durch ein Geräusch hörbar ist. Es kann ein Verschluss vorgesehen sein, damit die Vorrichtung insbesondere nach der Herstellung des Abdrucks des Fusses sicher verschlossen werden kann und der hergestellte Abdruck des Fusses, welcher sich innerhalb der Vorrichtung befindet, geschützt ist. Bei der Herstellung des Abdrucks des Fusses wird durch das spürbare Einrasten oder das Geräusch signalisiert, dass sich die Vorrichtung in der zweiten Konfiguration befindet, wobei gegebenenfalls eine definierte Zeit abzuwarten ist, bevor die Vorrichtung von der zweiten Konfiguration in die erste Konfiguration überführt wird, um den Fuss aus der Vorrichtung zu entfernen.

Bevorzugt umfasst die Vorrichtung wenigstens drei schwenkbar zueinander gelagerte Teile, vorzugsweise wenigstens vier schwenkbar zueinander gelagerte Teile, welche in die erste Konfiguration und in die zweite Konfiguration bringbar sind. Eine aus drei Teilen gebildete Vorrichtung kann beispielsweise aus einer Bodenplatte sowie einem linken und rechten Teil bestehen, wobei beispielsweise der linke Teil mit der Bodenplatte fest verbunden ist und der rechte Teil am linken Teil schwenkbar angebracht ist. Eine aus vier Teilen gebildete Vorrichtung kann beispielsweise aus einer Bodenplatte, einem linken und einem rechten Teil sowie einem Vorderteil bestehen, wobei beispielsweise der linke Teil mit der Bodenplatte fest verbunden ist und der rechte Teil am linken Teil schwenkbar angebracht ist sowie der Vorderteil an der Bodenplatte schwenkbar angebracht ist, wobei der linke und der rechte Teil beispielsweise vorgesehen sind, um einen unteren Abschnitt des Unterschenkels, den Knöchel, die Ferse und einen Abschnitt von der Ferse bis zum Mittelfuss aufzunehmen, und wobei der Vorderteil beispielsweise vorgesehen ist, um die Zehen und einen Abschnitt von den Zehen bis zum Mittelfuss aufzunehmen. Je nach gewünschten Anforderungen betreffend die Herstellung des Abdrucks des Fusses kann die Vorrichtung selbstverständlich aus mehr als vier zueinander schwenkbaren Teilen aufgebaut sein.

Bevorzugt sind eine Bodenplatte, ein linker Hinterteil, ein rechter Hinterteil und ein Vorderteil vorgesehen, wobei der linke Hinterteil an der Bodenplatte fest verankert ist, wobei der rechte Hinterteil am linken Hinterteil verschwenkbar angebracht ist, wobei der Vorderteil an der Bodenplatte verschwenkbar angebracht sind, wobei der linke und der rechte Hinterteil sich ergänzende Öffnungen aufweisen, sodass in der zweiten Konfiguration durch diese Öffnungen Platz für einen Unterschenkel geschaffen wird. Der linke und rechte Hinterteil sind vorgesehen, um einen Abschnitt des Unterschenkels, den Knöchel, die Ferse und einen Abschnitt von der Ferse bis zum Mittelfuss aufzunehmen. Der Vorderteil ist vorgesehen, um die Zehen und einen Abschnitt vom Mittelfuss bis zu den Zehen aufzunehmen. Durch die Öffnung im linken und rechten Hinterteil ist gewährleistet, dass der Fuss in den inneren Bereich der Vorrichtung bringbar ist, wobei ab einer bestimmten Höhe des Unterschenkels kein Druck angelegt ist und der Unterschenkel frei aus der Öffnung herausragt.

In einem Verfahren zur Herstellung eines Abdrucks eines Fusses mit Hilfe einer beschriebenen Vorrichtung wird die Vorrichtung, welche wenigstens zwei schwenkbar zueinander gelagerte Teile umfasst und in eine erste Konfiguration und in eine zweite Konfiguration bringbar ist, wobei sie in der zweiten Konfiguration eine kistenartige Form aufweist,
a) in einem ersten Schritt von einer ersten Konfiguration in eine zweite Konfiguration überführt, so dass in der zweiten Konfiguration ein Fuss in einen inneren Bereich der kistenartigen Form der Vorrichtung gebracht wird, und
b) in einem zweiten Schritt von der zweiten Konfiguration wieder in die erste Konfiguration überführt, wodurch der Fuss aus dem inneren Bereich der kistenartigen Form der Vorrichtung befreit wird.

Mit diesem Verfahren kann mit einer leicht transportierbaren Vorrichtung ein Abdruck eines Fusses hergestellt werden. Das Überführen der Vorrichtung von der erste Konfiguration in die zweite Konfiguration und zurück sind einfache Verfahrensschritte und ermöglichen eine schnelle und exakte Herstellung des Abdrucks des Fusses.

In einem Verfahren zur Herstellung von massangefertigten Schuhen
a) wird mit dem vorgängig beschriebenen Verfahren zur Herstellung eines Abdrucks eines Fusses ein Abdruck eines Fusses hergestellt,
b) dann wird aufgrund des Abdrucks des Fusses ein Leisten hergestellt,
c) und schliesslich wird aufgrund des Leistens ein massangefertigter Schuh hergestellt.

Das Verfahren zur Herstellung eines Abdrucks eines Fusses kann beispielsweise von einem Kunden direkt bei sich zu Hause selbständig durchgeführt werden, indem dem Kunden per Post eine Vorrichtung zugestellt wird, der Kunde das Verfahren zur Herstellung des Abdrucks des Fusses selbständig durchführt und dann die Vorrichtung mit dem hergestellten Abdruck des Fusses per Post versendet. Der Empfänger der Vorrichtung mit dem hergestellten Abdruck des Fusses kann anschliessend mit den Massen des Abdrucks des Fusses einen Leisten herstellen und aufgrund des Leistens einen massangefertigten Schuh herstellen. Der massangefertigte Schuh wird dem Kunden wieder per Post zugestellt, so dass der Kunde das Haus gar nicht verlassen muss, um einen passgenauen Schuh zu erhalten.

Vorzugsweise wird ein 3D-Scan des Abdrucks des Fusses erstellt, wobei der Leisten aufgrund des 3D-Scans hergestellt wird. Dazu können hochmoderne, festinstallierte Vorrichtungen wie ein 3D-Scanner und ein 3D-Drucker eingesetzt werden. Es ist auch denkbar, dass der Leisten nach der Herstellung wieder per Post an einen weiteren Empfänger versendet wird, welcher auf die eigentliche Schuhherstellung aufgrund eines Leistens spezialisiert ist. So können dem Kunden massangefertigte Schuhe zugestellt werden, welche auf spezialisierten, exakten und effizienten Produktionseinrichtungen hergestellt sind.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:

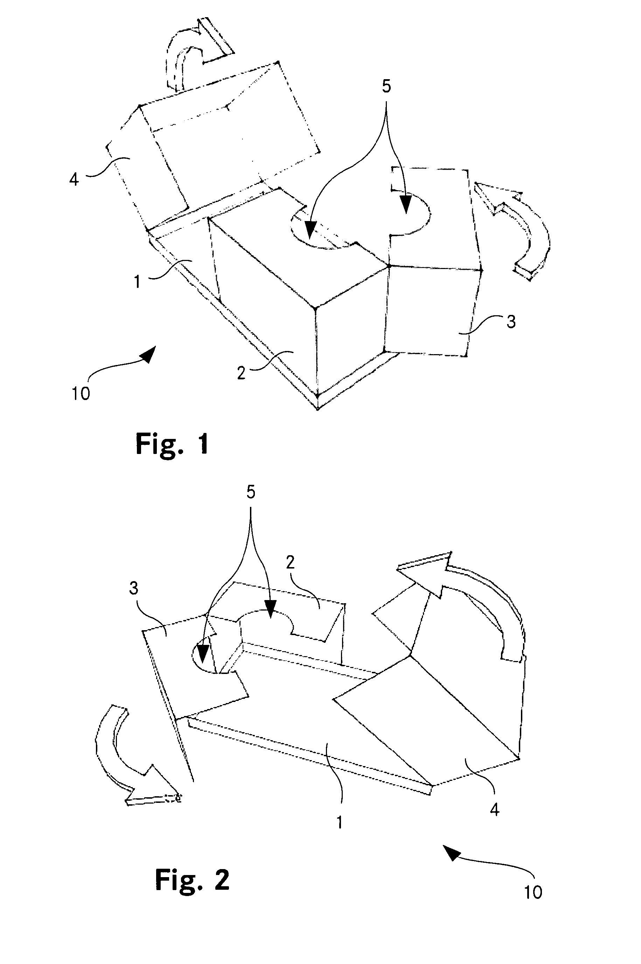

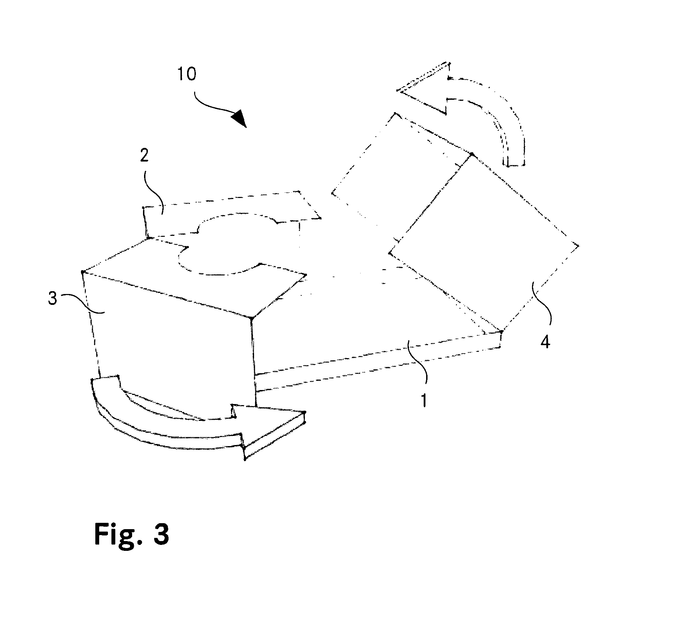

Fig. 1, 2, 3 drei perspektivische Ansichten einer erfindungsgemässen Vorrichtung zur Herstellung eines Abdrucks eines Fusses.

Grundsätzlich sind in den Figuren gleiche Teile mit gleichen Bezugszeichen versehen.

### Wege zur Ausführung der Erfindung

Fig. 1, 2, 3 zeigen drei perspektivische Ansichten einer erfindungsgemässen Vorrichtung 10 zur Herstellung eines Abdrucks eines Fusses aus drei verschiedenen Betrachtungspunkten. In den Fig. 1, 2, 3 ist die Vorrichtung 10 in der ersten Konfiguration dargestellt, d. h. die Vorrichtung 10 ist vorbereitet, damit ein Fuss in den inneren Bereich bringbar ist oder damit der Fuss aus der Vorrichtung befreibar ist.

Die zweite Konfiguration ist in Fig. 1, 2, 3 nicht dargestellt. Auch das Material zur Abnahme des Abdrucks des Fusses oder der Fuss sind in Fig. 1, 2, 3 nicht dargestellt.

Wie in Fig. 1, 2, 3 skizziert, weist die Vorrichtung 10 zur Herstellung eines Abdrucks eines Fusses eine Bodenplatte 1, einen linken Hinterteil 2, einen rechten Hinterteil 3 und einen Vorderteil 4 auf. Die Bodenplatte 1 ist rechteckig und dazu vorgesehen, dass eine Person einen Fuss darauf setzen kann, wobei genügend Platz vorhanden ist, um ein Material zur Abnahme eines Abdrucks des Fusses anzuordnen.

Der linke Hinterteil 2 weist drei senkrecht zueinander stehende Flächen auf, welche der Vorderfläche, der Seitenfläche und der Deckfläche eines Quaders entsprechen. Der Hinterteil 2 ist an der Bodenplatte fest angebracht, und zwar so, dass die unteren Kanten des Hinterteils 2 an eine Ecke der Bodenplatte 1, welche somit der linken hinteren Ecke entspricht, zu liegen kommt.

Der rechte Hinterteil 3 ist spiegelsymmetrisch zum linken Hinterteil 2 und über ein vertikales Scharnier am linken Hinterteil 2 angebracht. Der rechte Hinterteil 2 lässt sich somit nach rechts ausschwenken, um die Vorrichtung 10 in die erste Konfiguration zu bringen, und nach links zurückschwenken, um die Vorrichtung 10 in die zweite Konfiguration zu bringen.

Wie in Fig. 1, 2, 3 gezeigt, weisen der linke und der rechte Hinterteil 2, 3 an der Deckfläche halbkreisförmige Öffnungen 5 auf, welche in der zweiten Konfiguration eine kreisförmige Öffnung bilden. Somit kann der rechte Hinterteil 3 nach rechts ausgeschwenkt, ein Fuss auf die Bodenplatte gestellt und der rechte Hinterteil 3 nach links zurückgeschwenkt werden, wobei der Unterschenkel durch die Öffnungen 5 ragt.

Der linke und rechte Hinterteil überdecken im Wesentlichen die halbe Länge der rechteckigen Bodenplatte 1.

Die verbleibende Länge der rechteckigen Bodenplatte 1 wird vom Vorderteil 4 überdeckt. Der Vorderteil 4 weist vier senkrecht zueinander angeordnete Flächen auf, welche einem Quader mit fehlender Bodenfläche und einer fehlenden Seitenfläche entsprechen. Der Vorderteil 4 ist zwischen der der vorhandenen Seitenfläche entsprechenden Fläche mit einem Scharnier an der Bodenplatte 1 angebracht. Somit lässt sich der Vorderteil 4 aufklappen, um die Vorrichtung 10 in die erste Konfiguration zu bringen und der Vorderteil 4 lässt sich zuklappen, um die Vorrichtung 10 in die zweite Konfiguration zu bringen.

In der ersten Konfiguration ist somit der rechte Hinterteil 3 nach rechts ausgeschwenkt und der Vorderteil 4 ist aufgeklappt. In der zweiten Konfiguration liegt der rechte Hinterteil 3 symmetrisch am linken Hinterteil 2 an und der Vorderteil ist zugeklappt und liegt sowohl am linke als auch am rechten Hinterteil 2, 3 an. Wie aus Fig. 1, 2, 3 leicht ersichtlich ist, wird in der zweiten Konfiguration durch die Bodenplatte 1, den rechten und linken Hinterteil 2, 3 sowie den Vorderteil 4 eine leicht transportierbare kistenähnliche Form gebildet.

Die Bestandteile der Vorrichtung 10 können wie erwähnt aus Karton, Holz, Kunststoff, Metall oder irgendeinem anderen Material hergestellt sein.

In der Vorrichtung 10 ist ein Material zur Abnahme des Abdrucks des Fusses angeordnet. Dieses Material ist bevorzugt ein Hartschaum, welcher vollständig kompressibel und inelastisch ist.

Anstelle eines Vorderteils 4 können der linke und rechte Hinterteil 2, 3 die gesamte Bodenplatte überdecken, wobei der linke und rechte Hinterteil an den Vorderseiten je eine zusätzliche Fläche aufweisen, so dass in der zweiten Konfiguration ein geschlossener Körper mit einer kistenähnlichen Form gebildet ist.

Zusammenfassend ist festzustellen, dass eine Vorrichtung zur Herstellung eines Abdrucks des Fusses angegeben ist, welche leicht transportierbar ist, wobei ein Abdruck des Fusses schnell und exakt hergestellt werden kann.

## Patentansprüche

1. Vorrichtung (10) zur Herstellung eines Abdrucks eines Fusses, **dadurch gekennzeichnet,**
a) **dass** sie eine Aufnahme für den Fuss mit wenigstens zwei zueinander schwenkbar gelagerte Teile (1, 2, 3, 4) umfasst;
b) so dass die Aufnahme durch Verschwenken in eine erste Konfiguration und in eine zweite Konfiguration bringbar ist;
c) **dass** in der zweiten Konfiguration die wenigstens zwei Teile (1, 2, 3, 4) derart angeordnet sind, dass die Vorrichtung (10) eine kistenähnliche Form aufweist und dass sich Innenseiten der wenigstens zwei Teile (1, 2, 3, 4) in einem inneren Bereich der kistenähnlichen Form der Vorrichtung befinden;
d) **dass** durch eine Überführung der Vorrichtung (10) von der ersten Konfiguration in die zweite Konfiguration ein Fuss in den inneren Bereich der kistenähnlichen Form der Vorrichtung bringbar ist und
e) **dass** durch eine Überführung der Vorrichtung (10) von der zweiten Konfiguration in die erste Konfiguration der Fuss aus der kistenähnlichen Form der Vorrichtung (10) in der zweiten Konfiguration befreibar ist.

2. Vorrichtung (10) nach Anspruch 1, **dadurch gekennzeichnet, dass** durch eine Überführung der Vorrichtung von der ersten Konfiguration in die zweite Konfiguration der Fuss wenigstens bis zum Knöchel in den inneren Bereich der kistenähnlichen Form der Vorrichtung (10) bringbar ist.

3. Vorrichtung (10) nach einem der Ansprüche 1 bis 3, **dadurch gekennzeichnet, dass** die Vorrichtung (10) formstabil ausgeführt ist, so dass sie in der zweiten Konfiguration sowohl mit als auch ohne Fuss im inneren Bereich der kistenähnlichen Form bei äusserer Krafteinwirkung ihre Form beibehält.

4. Vorrichtung (10) nach einem der Ansprüche 1 bis 3, **dadurch gekennzeichnet, dass** mindestens zwei der wenigstens zwei Teile (1, 2, 3, 4) auf der Innenseite mit einem Material zur Abnahme des Abdrucks eines Fusses ausgekleidet sind.

5. Vorrichtung (10) nach Anspruch 4, **dadurch gekennzeichnet, dass** das Material zur Abnahme des Abdruck eines Fusses auf der Innenseite der mindestens zwei Teile (1, 2, 3, 4) derart geformt ist, dass in der zweiten Konfiguration der Fuss lückenlos durch das Material umschliessbar ist.

6. Vorrichtung (10) nach Anspruch 4 oder 5, **dadurch gekennzeichnet, dass** das Material zur Abnahme des Abdrucks eines Fusses vollständig kompressibel und inelastisch ist, wobei es sich vorzugsweise um einen Hartschaum handelt.

7. Vorrichtung (10) nach einem der Ansprüche 4 bis 6, **dadurch gekennzeichnet, dass** auf der Aufnahme und/oder dem Material zur Abnahme des Abdrucks eines Fusses eine Markierung für eine korrekte Positionierung des Fusses angebracht ist.

8. Vorrichtung (10) nach einem der Ansprüche 1 bis 7, **dadurch gekennzeichnet, dass** sie einen Mechanismus umfasst, welcher bei einer Überführung von der ersten Konfiguration in die zweite Konfiguration ein Erreichen der zweiten Konfiguration angibt, indem ein Einrasten spürbar oder durch ein Geräusch hörbar ist.

9. Vorrichtung (10) nach einem der Ansprüche 1 bis 8, **dadurch gekennzeichnet, dass** sie wenigstens drei schwenkbar zueinander gelagerte Teile (1, 2, 3, 4), vorzugsweise wenigstens vier schwenkbar zueinander gelagerte Teile (1, 2, 3, 4) umfasst, welche in die erste Konfiguration und in die zweite Konfiguration bringbar sind.

10. Vorrichtung nach einem der Ansprüche 1 bis 8, **dadurch gekennzeichnet, dass** eine Bodenplatte (1), ein linker Hinterteil (2), ein rechter Hinterteil (3) und ein Vorderteil (4) vorgesehen sind, wobei der linke Hinterteil (2) an der Bodenplatte (1) fest verankert ist, wobei der rechte Hinterteil (3) am linken Hinterteil (2) verschwenkbar angebracht ist, wobei der Vorderteil (4) an der Bodenplatte (1) verschwenkbar angebracht ist, wobei der linke und der rechte Hinterteil (2, 3) sich ergänzende Öffnungen (5) aufweisen, sodass in der zweiten Konfiguration durch diese Öffnungen (5) Platz für einen Unterschenkel geschaffen wird.

11. Verfahren zur Herstellung eines Abdruck des Fusses mit Hilfe einer Vorrichtung (10) nach einem der Ansprüche 1 bis 10, **dadurch gekennzeichnet, dass** die Vorrichtung (10), welche wenigstens zwei schwenkbar zueinander gelagerte Teile (1, 2, 3, 4) umfasst und in eine erste Konfiguration und in eine zweite Konfiguration bringbar ist, wobei sie in der zweiten Konfiguration eine kistenartige Form aufweist,
a) in einem ersten Schritt von einer ersten Konfiguration in eine zweite Konfiguration überführt wird, so dass in der zweiten Konfiguration ein Fuss in einen inneren Bereich der kistenartigen Form der Vorrichtung (10) gebracht wird, und
b) in einem zweiten Schritt von der zweiten Konfiguration wieder in die erste Konfiguration überführt wird, wodurch der Fuss aus dem inneren Bereich der kistenartigen Form der Vorrichtung (10) befreit wird.

12. Verfahren zur Herstellung von massangefertigten Schuhen, **dadurch gekennzeichnet,**
a) **dass** mit dem Verfahren nach Patentanspruch 11 ein Abdruck eines Fusses hergestellt wird,
b) **dass** aufgrund des Abdrucks des Fusses ein Leisten hergestellt wird,
c) **dass** aufgrund des Leistens ein massangefertigter Schuh hergestellt wird.

13. Verfahren nach Anspruch 12, **dadurch gekennzeichnet, dass** ein 3D-Scan des Abdrucks des Fusses erstellt wird, wobei der Leisten aufgrund des 3D-Scans hergestellt wird.
